(19) Europäisches Patentamt
European Patent Office
Office européen des brevets

(11) Publication number: **0 117 070**
**B1**

(12) # EUROPEAN PATENT SPECIFICATION

(45) Date of publication of patent specification: **29.08.90**

(51) Int. Cl.$^5$: **A 61 K 7/32**

(21) Application number: **84300373.2**

(22) Date of filing: **23.01.84**

(54) **Cosmetic sticks.**

(30) Priority: **26.01.83 US 460973**

(43) Date of publication of application:
**29.08.84 Bulletin 84/35**

(45) Publication of the grant of the patent:
**29.08.90 Bulletin 90/35**

(84) Designated Contracting States:
**AT BE CH DE FR GB IT LI NL**

(56) References cited:
**DE-A-2 352 266**
**FR-A-1 190 002**
**GB-A-2 062 466**
**US-A-2 942 008**
**US-A-3 641 239**
**US-A-4 126 679**
**US-A-4 151 272**
**US-A-4 229 432**
**US-A-4 280 994**

(73) Proprietor: **THE PROCTER & GAMBLE COMPANY**
**301 East Sixth Street**
**Cincinnati Ohio 45201 (US)**

(72) Inventor: **May, William George**
**4869 Beechwood Road**
**Cincinnati Ohio 45244 (US)**

(74) Representative: **Brooks, Maxim Courtney et al**
**Procter & Gamble (NTC) Limited Whitley Road**
**Longbenton**
**Newcastle-upon-Tyne NE12 9TS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Courier Press, Leamington Spa, England.

EP 0 117 070 B1

**Description**

The present invention relates to solid stick compositions, particularly solid antiperspirant sticks, having satisfactory strength and hardness while exhibiting aesthetically-pleasing attributes.

Many solid stick compositions are available in the art. Typically, these solid sticks are available in two basic forms. One such form is composed of soap/alcohol gel sticks, which may have stability problems. For instance, the combination of soap/alcohol gels with conventional astringent antiperspirant salts, such as aluminium chlorohydrate, can result in unstable gel structures rendering such sticks less aesthetically-pleasing to consumers.

In order to alleviate some of these problems, waxy materials, some in combination with volatile fluids, have been used in the development of solid stick compositions. US—A—4,126,679, November 21, 1978 to Davy and Drolet; US—A—4,229,432, October 21, 1980 to Geria; US—A—4,265,878, May 5, 1981 to Keil; US—A—4,280,994, July 28, 1981 to Turney disclose such compositions. The most commonly used waxy material in sticks of this type include long-chain fatty alcohols. Some use such alcohols in combination with volatile silicones. Although these fatty alcohol/volatile silicone solid sticks avoid some of the soap gel problems, they may vary unacceptably in structural hardness and strength. Therefore, formulations are sought which provide strong fatty alcohol/volatile silicone-based sticks without causing those sticks to become too brittle and cosmetically unacceptable.

Surprisingly, the addition of fatty alcohols, $C_{20}$ and longer, produces such a product. That is, not only is the strength of such a stick increased, but that strength enhancement does not result in overly hard, brittle product.

It is an object, therefore, of the present invention to provide solid stick compositions containing long-chain higher fatty alcohols and a volatile silicone wherein such compositions contain sufficient $C_{20}$ alcohol and longer chain alcohols to result in a stable and strong final product. It is a further object of the present invention to not only provide strong final products but to provide aesthetically-pleasing products as well. Another object of this invention is to provide solid stick compositions as described above which additionally contain astringent antiperspirant salt materials. These and other objectives will become more apparent from the following disclosure. All percentages and ratios herein are by weight unless otherwise designated.

According to the present invention, there is provided a solid antiperspirant stick composition possessing improved strength without undue brittleness comprising:

(a) from 5% to 20% by weight of a long-chain fatty alcohol having from 8 to 18 carbon atoms in its chain;

(b) from 35% to 55% by weight of a cyclic or linear volatile polydimethylsiloxane having from 3 to 9 silicon atoms in its chain; and

(c) from 10% to 70% by weight of an astringent antiperspirant salt;

wherein said composition contains an additional long chain fatty alcohol selected from $C_{20}$ alcohol, alcohols whose chains are longer than $C_{20}$ and may go up to $C_{26}$ and mixtures thereof at a level of from 1% to 3% by weight of the total fatty alcohol level.

The present solid stick compositions contain certain essential and may in addition contain other nonessential, optional components. Each component is discussed in detail below.

Long-chain Higher Fatty Alcohol

The higher fatty alcohols of the compositions of the present invention include as a first component a fatty alcohol containing from 8 to 18 carbon atoms in its chain, preferably from 12 to 18 carbon atoms. Examples suitable for use in the invention at hand include cetyl alcohol, stearyl alcohol, myristyl alcohol, lauryl alcohol, and mixtures thereof.

From 5% to 20% higher fatty alcohol is present in the solid stick compositions, preferably from 10% to 15%.

As indicated above, the compositions described herein also contain from 1% to 3%, based on the total weight of the long chain fatty alcohols present (the $C_{12}$—$C_{18}$ alcohols as well as the longer chain alcohols), of alcohols having chain lengths of $C_{20}$ or longer. A preferred alcohol of this type is commercially available $C_{20}$ alcohol which is a mixture of higher fatty alcohols wherein about 50% is $C_{20}$, about 30% is $C_{22}$, with the remainder primarily composed of alcohol chains longer than $C_{22}$. The alcohols useful in the present invention may go up to $C_{26}$ but it is preferred to have the majority in the $C_{20}$—$C_{22}$ range. The level of $C_{20}$ and longer chain alcohols found in the commercially available shorter long-chain fatty alcohols (e.g. stearyl alcohol) generally does not exceed about 0.8%. Only by increasing the level of these alcohols to from 1% to 3%, preferably from 1% to 2%, of the total higher fatty alcohol content will a stronger product result.

Volatile Silicone

The volatile silicones useful in the solid stick compositions of the present invention are either cyclic or linear polydimethylsiloxanes containing from 3 to 9, preferably from 4 to 5, silicon atoms.

The following formula illustrates the cyclic volatile polydimethylsiloxanes useful in the solid stick compositions disclosed herein:

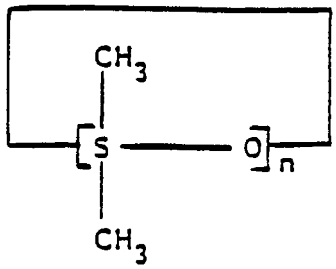

wherein n = 3 to 7.

The linear polydimethylsiloxanes contain from 3 to 9 silicon atoms per molecule and have the following general formula:

$$(CH_3)_3Si—O—(Si(CH_3)_2—O)_n—Si(CH_3)_3$$

wherein n = 1 to 7.

Examples of silicones of the above type include those offered by Dow Corning Corporation, Dow Corning 344, 345 and 200 fluids, Union Carbide, Silicone 7207 and Silicone 7158, and Stauffer Chemical, SWS-03314.

The linear volatile materials generally have viscosities of less than about 5 centistokes at 25°C while the cyclic materials have viscosities less than about 10 centistokes. "Volatile" means that the material has a measurable vapor pressure. A description of volatile silicones is found in Todd and Byers, "Volatile Silicone Fluids for Cosmetics", *Cosmetics and Toiletries,* Vol. 91, January, 1976, pp. 27—32.

The amount of volatile silicone used in the present compositions is from 35 to 55, preferably from 40 to 50%.

Optional Components

The solid stick compositions of the present invention may contain a variety of optional, nonessential components. These components serve a variety of functions such as improving the stability, cosmetics and/or aesthetics of the present compositions.

Wax

A preferred optional component includes a wax such as castor wax, fatty acids, silicone waxes and glycerol monostearate, and mixtures thereof at levels of from 1% to 10%, preferably from 3% to 10%. If present, the wax is believed to enhance structural stability at higher temperatures.

Emollient

The emollient component of the present invention is useful in providing an aesthetically-pleasing product. One type of emollient suitable for use in the present solid stick compositions is an ethylene oxide and/or propylene oxide condensation product having the following formula:

$$RO(C_2H_4O)_a(C_3H_6O)_bH$$

wherein R is either hydrogen or a hydrocarbon chain having from about 2 to 20 carbon atoms, preferably from about 4 to about 18, a and b are each from about 0 to about 35, and a + b is from about 5 to about 35.

Examples of such emollients include Fluid AP® (a condensate of about 14 moles of propylene oxide with about 1 mole of butyl alcohol sold by Union Carbide); a polypropylene glycol having molecular weight of about 1200; a polyethylene glycol having molecular weight of about 420; a condensate of 20 moles ethylene oxide and 5 moles propylene oxide with one mole of cetyl alcohol, and mixtures thereof.

Other emollients suitable for use in the present solid stick compositions include fatty acid and fatty alcohol esters and water insoluble ethers such as those disclosed in US—A—4,202,879, May 13, 1980 to Shelton, and mixtures thereof.

If present, the emollient is used at levels of from 1% to 10%, preferably from 3% to 7%.

Astringent Antiperspirant

Any aluminum astringent antiperspirant salt or aluminum and/or zirconium complex can be employed herein when the present sticks are intended for use as an antiperspirant. Useful are such salts as aluminum halides, aluminum hydroxyhalides, zirconyl oxyhalides, zirconyl hydroxyhalides, and mixtures thereof. The aluminum salts of this type include aluminum chloride and aluminum hydroxyhalide having the following formula:

$$Al_2(OH)_aCl_b \cdot XH_2O$$

a is from about 2 to about 5;

a + b = 6 and a and b need not be integers; and

x is from about 1 to about 6.

The manner of preparing such salts is disclosed in US—A—3,887,692, June 3, 1975 to Gilman.

Zirconium salts are also useful in the present invention. Such salts include zirconium oxychloride,

zirconium hydroxychloride, and zirconium salts of the formula $ZrO(OH)_{2-a}Cl_a \cdot nH_2O$, wherein a is from about 1.5 to about 1.87 and n is from about 1 to about 7. These types of zirconium salts are discussed in BE—A—825,146, August 4, 1975 to The Procter and Gamble Company.

Complexes of the previously-discussed astringent salts are disclosed in US—A—3,679,068, February 12, 1974 to Leudders, et al. The Leudders et al patent discloses complexes of aluminum, zirconium and glycine commonly known as ZAG complexes.

The preferred aluminum salt for use in such ZAG complexes is aluminum chlorhydroxide with the preferred zirconium compounds being zirconyl hydroxychloride and other zirconyl hydroxyhalides of the following formula:

$$ZrO(OH)_{2-a}Cl_a \cdot nH_2O$$

wherein a is from about 1.5 to about 1.87 and n is from about 1 to about 7. US—A—4,120,948, October 17, 1978 to Shelton discusses these preferred ZAG compounds.

Other antiperspirant complexes are also suitable for use in the present compositions. For example, US—A—3,903,258, September 2, 1975 to Siegel, discloses a zirconyl chloride/aluminum hydroxide and aluminum chlorhydroxide complex. US—A—3,979,510, September 6, 1976 to Rubino discloses complexes composed of particular aluminum, zirconium and aluminum buffer compounds. Additionally, US—A—3,981,986, September 21, 1976 to Rubino, discloses the aluminum and zirconium complexes with an organic buffer, and US—A—3,970,748, July 20, 1976 to Mecca, discloses an aluminum chlorhydroxy glycinate complex.

The levels of these antiperspirant materials range from 10% to 70%, preferably from 15% to 50%, most preferably from 15% to 25% on a pure antiperspirant salt basis. In the case of ZAG type materials glycine is excluded when antiperspirant salt level is determined.

Others

Another optional component of the present invention is inert filler material. This filler material enhances the aesthetic characteristics of the present solid sticks and may serve to stabilize the structure of such sticks.

Among the filler materials suitable for use in the present invention are talc, colloidal silica such as CAB—O—Sil (Cabot. Corp.), clays such as bentonite, and mixtures thereof. The inert filler material comprises from about 0.5% to about 10% of the present solid stick compositions.

Conventional deodorant materials also may be included in the present invention. Suitable deodorants include bacteriostatic quaternary ammonium compounds such as cetyl-trimethylammonium bromide, cetyl pyridinium chloride, benzethonium chloride, diisobutyl phenoxy ethoxy ethyl dimethyl benzyl ammonium chloride, sodium N-lauryl sarcosine, sodium N-palmethyl sarcosine, lauroyl sarcosine, N-myristoyl glycine, potassium N-lauryl sarcosine, stearyl, trimethyl ammonium chloride and mixtures thereof. Another suitable deodorant material is 2,4,4'-trichloro-2'-hydroxydiphenyl ether. If present, these deodorants comprise from about 0.1% to about 1.0% of the composition.

Other optional components include perfumes, pigments, dyes, coloring agents, and the like, at levels individually of from about 0.1% to about 2.0%.

Method of Manufacture

The solid stick compositions of the present invention are made in accordance with well-established methods known to those knowledgeable in the art.

Industrial Application

The present solid stick compositions are useful as vehicles for a wide variety of cosmetic materials. For instance, a preferred use of these solid stick compositions is as antiperspirant sticks. However, these sticks can be used as deodorant sticks as well as other cosmetic vehicles.

The following example is presented as an illustration of the present invention and is not limitative thereof.

Example I

An antiperspirant stick was made utilizing the following components:

| Component | Weight % |
|---|---|
| Cyclomethicone | 43.95 |
| Fluid AP | 4.99 |
| Stearyl Alcohol | 11.49 |
| Castor Wax | 4.99 |
| Talc | 6.99 |
| Zirconium/Aluminum/Glycine complex | 26.67 |
| Fragrance Masking Agent | 0.80 |
| $C_{20}OH$* | 0.12 |

*This is the commercial $C_{20}$ alcohol mixture described previously.

Example II

An antiperspirant stick similar to that described in Example I was prepared but the $C_{20}$ and longer chain alcohol level was only 0.034%. This stick therefore did not have the required amount of such long chain alcohols.

Example III

The sticks of Examples I and II were compared for softness and break strength. The softness measurements were made using a penetrometer while the break strength measurements were made using a Chatillon break strength tester. The results of these tests are shown below.

| | Penetration (Tenths of a mm)* | Break Strength (Kg (Pounds) Force)** |
|---|---|---|
| Example I Product | 78.1 | 5.1 (11.3) |
| Example II Product | 96.1 | 3.2 (7.1) |

*The penetration values are determined by using ASTM Method D-5. The measurements are made at least 10 mm from the edge of a stick.

**The breakstrength is determined using a Hunter Force Gauge, Model L-10, 0—13.6 Kg (0—30 lb) range. The gauge is attached to a slide which allows the gauge to contact the test stick through a breaker bar at a speed of 7.6 cm (three inches) per minute. The value recorded is the force gauge reading when the stick breaks.

It is seen that the product of the present invention has a greater break strength while also having a lower penetration value.

**Claims**

1. A solid antiperspirant stick composition possessing improved strength without undue brittleness comprising:

(a) from 5% to 20% by weight of a long-chain fatty alcohol having from 8 to 18 carbon atoms in its chain;

(b) from 35% to 55% by weight of a cyclic or linear volatile polydimethylsiloxane having from 3 to 9 silicon atoms in its chain; and

(c) from 10% to 70% by weight of an astringent antiperspirant salt;

characterized in that said composition contains an additional long chain fatty alcohol selected from $C_{20}$ alcohol, alcohols whose chains are longer than $C_{20}$ and may go up to $C_{26}$ and mixtures thereof at a level of from 1% to 3% by weight of the total long chain fatty alcohol level.

2. A solid stick composition according to Claim 1 wherein it additionally contains from 1% to 10% of a wax selected from castor wax, fatty acids, silicone waxes, glycerol monostearate, and mixtures thereof.

3. A solid stick composition according to Claim 1 or 2 wherein said $C_8$—$C_{18}$ long-chain fatty alcohol is present at a level of from 10% to 15% by weight and is selected from cetyl alcohol, stearyl alcohol, myristyl alcohol, lauryl alcohol, and mixtures thereof.

4. A solid stick composition according to any of Claims 1 to 3 which additionally contains from 1% to 10% of an emollient.

5. A solid stick composition according to Claim 4 wherein said emollient is selected from ethylene oxide and/or propylene oxide condensation products, fatty acid esters, fatty alcohol esters, water insoluble ethers, and mixtures thereof.

6. A solid stick composition according to Claim 1 wherein said astringent antiperspirant salt is a complex of aluminum chlorhydroxide, a zirconium compound and glycine.

7. A solid stick composition according to any of Claims 1 to 6 wherein said $C_{20}$ and/or longer chain alcohols are present at from 1% to 2% of the total long-chain fatty alcohol.

**Patentansprüche**

1. Feste, schweißhemmende Stiftzusammensetzung, welche eine verbesserte Festigkeit ohne übermäßige Sprödigkeit besitzt, umfassend:

(a) von 5 Gew.-% bis 20 Gew.-% von einem langkettigen Fettalkohol mit 8 bis 18 Kohlenstoffatomen in seiner Kette;

(b) von 35 Gew.-% bis 55 Gew.-% von einem cyclischen oder linearen, flüchtigen Polydimethylsiloxan mit 3 bis 9 Siliciumatomen in seiner Kette; und

(c) von 10 Gew.-% bis 70 Gew.-% von einem adstringierenden schweißhemmenden Salz;

dadurch gekennzeichnet, daß die genannte Zusammensetzung einen zusätzlichen langkettigen Fettalkohol, ausgewählt unter $C_{20}$-Alkoholen, Alkoholen, deren Ketten länger als $C_{20}$ sind und bis zu $C_{26}$ erreichen können, und Gemischen hievon, in einer Menge von 1 Gew.-% bis 3 Gew.-% der Gesamtmenge von langkettigem Fettalkohol, enthält.

2. Feste Stiftzusammensetzung nach Anspruch 1, worin sie zusätzlich von 1% bis 10% eines Wachses, ausgewählt unter Rizinuswachs, Fettsäuren, Silikonwachsen, Glycerinmonostearat und Gemischen hievon, enthält.

3. Feste Stiftzusammensetzung nach Anspruch 1 oder 2, worin der genannte langkettige $C_8$—$C_{18}$ Fettalkohol in einer Menge von 10 Gew.-% bis 15 Gew.-% vorliegt und unter Cetylalkohol, Stearylalkohol, Myristylalkohol, Laurylalkohol und Gemischen hievon ausgewählt ist.

4. Feste Stiftzusammensetzung nach einem der Ansprüche 1 bis 3, welche zusätzlich von 1% bis 10% eines Emolliens enthält.

5. Feste Stiftzusammensetzung nach Anspruch 4, worin das genannte Emolliens unter Ethylenoxid-und/oder Propylenoxidkondensationsprodukten, Fettsäureestern, Fettalkoholestern, wasserunlöslichen Ethern und Gemischen hievon ausgewählt ist.

6. Feste Stiftzusammensetzung nach Anspruch 1, worin das genannte adstringierende schweißhemmende Salz ein Komplex aus Aluminiumchlorhydroxid, einer Zirconiumverbindung und Glycin ist.

7. Feste Stiftzusammensetzung nach einem der Ansprüche 1 bis 6, worin die genannten $C_{20}$— und/oder längerkettigen Alkohole in einer Menge von 1% bis 2% der Gesamtmenge von langkettigem Fettalkohol vorliegen.

**Revendications**

1. Composition de stick antitranspirant solide possédant une résistance mécanique améliorée sans cassant exagéré, comprenant:

(a) de 5% à 20% en poids d'un alcool gras à chaîne longue comportant de 8 à 18 atomes de carbone dans sa chaîne;

(b) de 35% à 55% en poids d'un polydiméthylsiloxane volatil cyclique ou linéaire comportant de 3 à 9 atomes de silicium dans sa chaîne; et

(c) de 10% à 70% en poids d'un sel antitranspirant astringent;

caractérisée en ce que ladite composition contient un alcool gras à chaîne longue supplémentaire choisi parmi un alcool en $C_{20}$, les alcools dont les chaînes sont plus longues que $C_{20}$ et peuvent aller jusqu'à $C_{26}$ et leurs mélanges, à raison de 1% à 3% en poids de la proportion totale d'alcools gras à chaîne longue.

2. Composition de stick solide selon la revendication 1, contenant en outre de 1% à 10% d'une cire choisie parmi la cire de ricin, les acides gras, les cires de silicone, le monostéarate de glycérol et leurs mélanges.

3. Composition de stick solide selon la revendication 1 ou 2, dans laquelle ledit alcool gras à chaîne longue en $C_8$—$C_{18}$ est présent à raison de 10% à 15% en poids et est choisi parmi l'alcool cétylique, l'alcool stéarylique, l'alcool myristylique, l'alcool laurylique et leurs mélanges.

4. Composition de stick solide selon l'une quelconque des revendications 1 à 3, qui contient en outre de 1% à 10% d'un émollient.

5. Composition de stick solide selon la revendication 4, dans laquelle ledit émollient est choisi parmi les

produits de condensation d'oxyde d'éthylène et/ou d'oxyde de propylène, les esters d'acides gras, les esters d'alcools gras, les éthers insolubles dans l'eau et leurs mélanges.

6. Composition de stick solide selon la revendication 1, dans laquelle ledit sel antitranspirant astringent est un complexe de chlorohydroxyde d'aluminium, d'un composé du zirconium et de glycine.

7. Composition de stick solide selon l'une quelconque des revendications 1 à 6, dans laquelle lesdits alcools en $C_{20}$ et/ou à chaîne longue sont présents à raison de 1% à 2% de l'alcool gras à chaîne longue total.